Europäisches Patentamt

European Patent Office     (11) Publication number:    **0 223 280**
                                                         **A1**
Office européen des brevets

(19)

## (12) EUROPEAN PATENT APPLICATION

(21) Application number: **86201842.1**         (51) Int. Cl.⁴: **C 02 F 3/28**

(22) Date of filing: **23.10.86**

(30) Priority: **24.10.85 NL 8502914**          (71) Applicant: **Bakens, Johannes, Gerardus, M, Langenakker 20, NL-5731 JS Mierlo (NL)**

(43) Date of publication of application: **27.05.87 Bulletin 87/22**

(84) Designated Contracting States: **AT BE DE ES FR GR NL SE**    (72) Inventor: **Bakens, Johannes, Gerardus, M, Langenakker 20, NL-5731 JS Mierlo (NL)**

(54) **Multistage watertreatment system for wastewater under anaerobe conditions.**

(57) A process for the separate anaerobic treatment of sludge and waste water. The influent (6) is separated in a first apparatus (1), under anaerobic conditions, into an aqueous phase and a sludge phase. The settled sludge phase (5) is fed via a pipe (11) to a second apparatus (2) in which anaerobic digestion of the sludge takes place. The aqueous phase from the separator (1) is led (9) to a third apparatus (3) divided into a fermentation compartment (10) and a sedimentation compartment (20).

The treated water leaves the installation at (26) after an aeration (not shown).

The biogas produced in the fermenter (2) is used for mixing in the fermenter (10), where as the gas leaning the latter can be used in assisting the separation in the separator (1).

J.G. Bakens, Mierlo

Title: Method for the treatment of waste water under anaerobic conditions and an installation for this purpose.

The invention relates to the treatment of waste water under anaerobic conditions accompanied by the formation of biogas, sludge, purified waste water (in particular dischargeable waste water) and an installation for this purpose.

Waste water is understood to mean waste water released during the processing of vegetable or animal material, both on an industrial scale, for example in the production of sugar, starch, paper, fruits and gelatine, and on a smaller scale, as sometimes takes place on farms, in the bio industry and in smaller or larger residential units, e.g. hotels, for the processing of faeces.

It is known that waste water can be fermented under anaerobic conditions and further processed in such a way that biogas, sludge (or compost) and purified waste water results.

A particular problem in this respect is the degree of purity of the processed waste water because, depending on the government regulations, this determines whether the purified waste water may or may not be discharged on surface water.

The known processes for the anaerobic treatment of waste water mainly differ in their objective, namely whether the main aim of the process is to obtain biogas, sludge (or compost) and purified waste water, or a combination of these.

In a known method and installation for the treatment of waste water a so-called sludge phase is transported to a fermenter in which an anaerobic fermentation of the organic material takes place accompanied by the formation of biogas, after which the total content of the fermenter, consisting of water, micro-organisms and wholly or partly fermented organic material is passed on to what is termed an after-fermenter. Next, the liquid phase is often separated from the sludge which has settled and ceased fermenting and passed on, possibly after prior filtration, to a lava bed for aeration before the aqueous phase is discharged on to surface water.

As a rule, the separated sludge is transported to a concentrator for the formation of compost, although the sludge obtained can already be used as compost. In this known method biogas as well as compost, and possibly also dischargeable purified waste water, are produced.

The disadvantage of this method is the long period of time for which the material to be fermented remains both in the fermenter and in the after-fermenter. Depending on the temperature at which the fermentation takes place, this time varies from a period of about twenty to sixty days.

Since the size of the installation at a constant supply of the waste water to be processed is proportional to the dwell times in the fermentation rooms, the known installations are relatively large and hence expensive.

The object of the present invention is to provide a method and an installation intended for its application in which the treatment of waste water by the conversion into biogas, sludge and purified waste water can take place considerably more quickly than in known installations.

In what follows below, the influent is understood to mean the waste water brought up, which is subjected to the method in accordance with the invention. The influent will frequently be brought up from a buffer zone or storage area, in which the waste water to be treated is collected.

Depending on the circumstances the influent may be a sludge phase deposited in a buffer zone, an aqueous phase which has been separated in a buffer zone, or a mixture of these, or it may be the waste water from a buffer zone that has not (yet) been separated into an aqueous phase and a sludge phase.

If no buffer zone is used influent is understood to mean waste water which has been brought up.

The invention consists of a method for the treatment of waste water by anaerobic fermentation in a number of successive operations for the preparation of biogas, sludge and purified waste water and is characterised in that the influent is separated under anaerobic conditions into an aqueous phase and a sludge phase and that each phase is subjected separately to an individual anaerobic fermentation, accompanied by the formation of biogas, sludge and purified waste water, in which respect the water phase which occurs during the fermentation of the sludge phase is conveyed to the above-mentioned aqueous phase.

The invention is based on the situation occurring in practice that waste water which becomes available either regularly or irregulary is collected in buffer zones from which it is drawn off for treatment.

In accordance with the invention, the influent is brought into a first arrangement closed off from the air for the separation into an aqueous phase and a sludge phase.

Next this sludge phase, possibly after combination with the sludge phase which has settled in the buffer zone, if present, is brought via a pipe to a second arrangement closed off from the air where an anaerobic fermentation of the sludge takes place.

In addition, the aqueous phase of the first arrangement is led via a pipe to a third arrangement, likewise closed off from the air, into which the water phase separated in the second arrangement also flows, for the anaerobic fermentation of the solid constituents of the collected phases.

To facilitate and/or accelerate the separation of the aqueous phase and the sludge phase in the first arrangement it may be advantageous to add flotation and/or sedimentationpromoting agents to the aqueous phase which comes from the buffer zone, if present. This can also already be done in the buffer zone, if present.

In order to promote the separation between the aqueous and the sludge phase in the first arrangement it is advantageous for the first arrangement it is advantageous for the first arrangement to be provided with means for collecting and removing sludge which is sinking as a result of sedimentation, and with a means for collecting and removing sludge floating upwards as a result of flotation. For preference, the sludge separated by sedimentation and flotation is led collectively to the second arrangement.

In developing the invention it was found that it is advantageous if biogas is captured and retained by sludge rising upwards in the first arrangement and is carried to the second arrangement along with the sludge removed. It was found that this gasification with biogas promotes the anaerobic fermentation in the second and in the third arrangement.

For the same reason it is advantageous to gasify with biogas the sludge which has sunk in the first arrangement before it is subjected to fermentation in the second arrangement.

The biogas formed in another stage of the process is, of course, used for this gasification.

Whilst according to the method of the invention the first arrangement is mainly used for separating the influent from a buffer zone into an aqueous and a sludge phase under anaerobic conditions, the intention of the second arrangement is to subject the sludge phase to a main fermentation.

Needless to say, the fermentation is conducted in such a way that at the end of the process in the second arrangement as few fermentable constituents as possible collect.

With the formation of the sediment in the second arrangement a water phase occurs in it. Apart from particles which have not yet been separated from the sludge phase to be fermented, this also contains fermentable particles and micro-organisms which bring about the fermentation.

In accordance with the invention this water phase is added to the aqueous phase which leaves the first arrangement in order to convey this, either collectively or separately, to the third arrangement in order to allow as much fermentable material as possible to ferment.

In this third arrangement a fermentation of the liquid takes place. Known fermenters can be used for this purpose.

It has proved advantageous for the speed of fermentation in the third arrangement to keep the combined aqueous phase and water phase in motion during fermentation. For preference, this agitation should be done by injecting biogas formed during the fermentation. It has proved that this measure has a greater accelerating effect on the fermentation than others.

The fermented water phase which leaves the third arrangement has been extensively purified of organic fermentable material compared with the influent used as starting material in the method according to the invention.

For example, according to the method of the invention it has proved possible to achieve a degree of purification of more than 98%. This percentage compares favourably with that of other processes in which no more than 90% is achieved as a rule. Since the process in the invention can be conducted in such a way that optimum fermentation occurs, an optimum production of biogas can also be realised according to the method of the invention.

Needless to say, all the biogas formed in the various arrangements of the installation according to the invention must then be captured and collected, possibly after first being led back either wholly or partly into the arrangements in order to support the fermentation.

It can be pointed out that the influent may contain impurities that do not originate from the animal or vegetable material, during the processing of which the influent has formed, as long as these impurities do not disturb the anaerobic fermentation.

Since the fermented water phase which leaves the third arrangement (also sometimes termed the effluent) contains no oxygen, it must not be discharged on to the surface water without further treatment. The effluent is made suitable for this by aeration.

The invention also relates to an installation which is intended for the application of the method of the invention.

This part of the invention consists of a number of arrangements closed off from the air for the anaerobic fermentation in biogas, sludge and purified waste water which is characterised in that the installation comprises a first arrangement equipped with a supply pipe for influent from a buffer zone, with means for the separation into an aqueous phase and a sludge phase, which first arrangement is also equipped with separate discharge pipes for the separate removal of biogas, the aqueous phase and sludge phase which have formed during the stay in the first arrangement, in which respect the discharge pipe of the sludge phase formed in the first arrangement leads to a second arrangement closed off from the air for the anaerobic fermentation of the sludge phase, in which respect the discharge pipe may possibly be joined to the discharge pipe of the sludge phase from the buffer zone for the waste water, which second arrangement is also equipped with separate discharge pipes for the biogas formed, the fermented sludge which has settled and a water phase which has formed, in which respect the discharge pipe for the water phase formed from the second arrangement, in combination with the discharge pipe of the aqueous phase from the first arrangement, leads to a third arrangement closed off from the air for the anaerobic ferementation of the collected water and aqueous phases, which third arrangement is also equipped with separate discharge pipes for the biogas formed, the purified waste water and the fermented sludge that has settled in the third arrangement.

According to the preferred system of the invention the installation also contains systems for leading biogas through the influent from the first arrangement and/or through the water/aqueous phase in the third arrangement and for conveying settled, fermented sludge to the sludge phase to be fermented in the second arrangement.

For preference, the third arrangement in the installation according to the invention consists of at least two inter-connected compartments, the first compartment of which contains means for keeping the aqueous phase brought up in motion and the last compartment contains means for promoting the separation of settled, fermented sludge.

For preference, however, the third arrangement consists of two compartments: an agitation space and a sedimentation space, in which respect means are present in the first space for passing biogas through.

The method and the installation according to the invention are further explained on the basis of a schematic representation (drawing 1) of an arrangement according to the invention, which explanation, for that matter is not intended to restrict the invention.

In this, a buffer zone for the waste water is indicated by 0 and the first, second and third arrangements indicated in the description are referred to by 1,2 and 3, respectively.

Arrangement 1 contains a flotation/sedimentation space 4 and a sludge hopper 5 and openings not further indicated for the supply of influent via supply pipe 6 from the buffer zone and for the discharge of the gas formed via pipe 7 to a compressor 8, for the discharge of the aqueous phase formed via pipe 9 to the agitation space 10 located on the left in the arrangement 3 and for the discharge of sludge from the sludge hopper 5 via pipes 11 and 14 to the arrangement 2.

In the diagram, pipe 11 is joined to pipe 12 which brings up sludge from the buffer zone.

Arrangement 2 is a fermentation arrangement. Consideration can be given to using a normal fermenter for this purpose.
Arrangement 2 contains a number of partitions in order to effect a separation between the collected sludge flows from pipes 11 and 12 brought up with pipe 14, combined with the sludge phase supplied from arrangement 3 and the gas flow removed with pipe 17.

In addition, arrangement 2 contains a separation arrangement 18, for example, a sloping bottom for bringing the fermented sludge to a discharge chute or opening. In addition, arrangement 2 contains openings which are not further indicated for bringing up sludge via pipe 14, and for the removal of gas (pipe 17), to the compressor, of the water phase to arrangement 3 (pipe 16) and fermented sludge (pipe 19).

Arrangement 3 is a fermentation arrangement. Consideration can be given to using a normal fermentation arrangement for this purpose.

Arrangement 3 is divided into two compartments 10 and 20 which are connected to each other.

Compartment 10 contains an installation 21 for bringing the liquid into motion in the form of a system of pipes with openings from which gas flows, which is conveyed through pipe 22 via the compressor 8. Compartment 20 is a sedimentation space which is equipped with means 23 for capturing and collecting sediment in the form of sludge in order, via pipe 15, to remove it via pipe 14 to arrangement 2 (fermenter).

In addition, arrangement 3 is equipped with openings for bringing the water/aqueous phase via pipe 24 to compartment 10, for the removal of gas 25 from compartment 20 to the compressor 8, and for the removal of purified waste water 26 (effluent) from compartment 20. As a rule, the effluent delivered by the installation, after adequate aeration, is immediately suitable for discharge on to surface water, because the process can be carried out in such a way that 98% or more of the impurities originally present in the influent are removed from the liquid phase in the form of sludge, all this being accompanied by the formation of gas.

Since the anaerobic fermentation is used separately and individually on the separate flows of the sludge phase and the water/aqueous phase delivered from the influent, the anaerobic treatment process takes place much more quickly than is the case with related, known processes in which under otherwise comparable conditions a separation of this kind is not carried out.

The gas produced by the arrangement according to the invention, which mainly consists of lower hydrocarbons, is an excellent source of energy.

The sludge removed can be processed into compost in a way which is known in itself and is eminently suitable as a soil improvement agent.

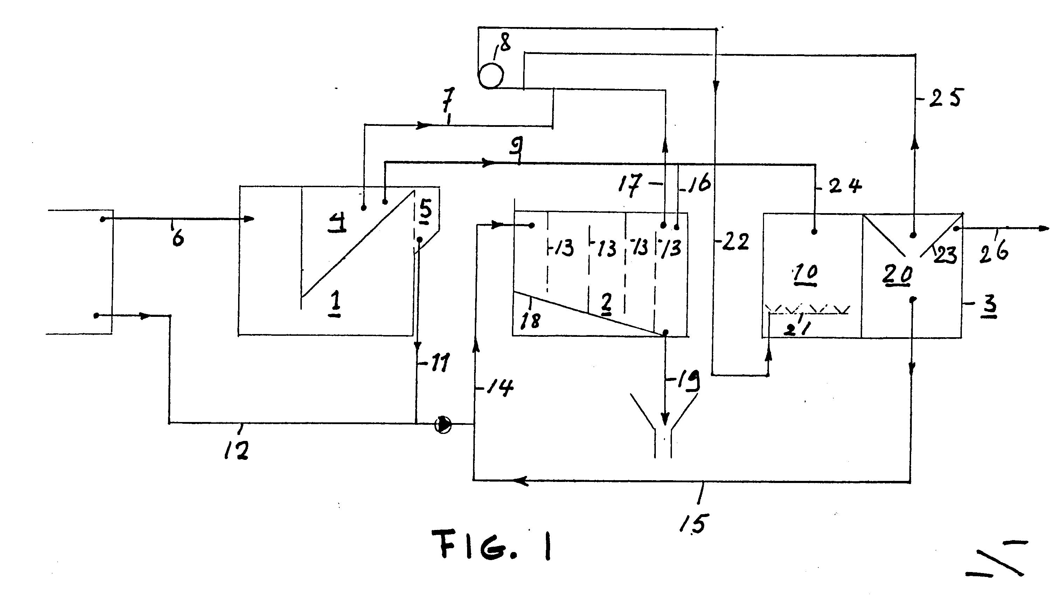

Claims

1. Method for the treatment of waste water by anaerobic purification in a number of successive operations for the preparation of biogas, sludge and purified waste water, characterised in that the influent is separated under anaerobic conditions into an aqueous phase and a sludge phase and that each phase is subjected separately to an individual anaerobic fermentation, accompanied by the formation of biogas, in which respect the water phase which occurs during fermentation of the sludge phase is conveyed to the above-mentioned aqueous phase and the collected aqueous phase.

2. Method in accordance with claim 1, characterised in that the influent, from a buffer zone, is brought up to a first arrangement for the separation under anaerobic conditions into an aqueous phase and a sludge phase, in which respect the sludge phase formed, possibly in combination with the sludge phase brought up from the buffer zone, is next led to a second arrangement for the anaerobic fermentation of the sludge brought up and in which respect the aqueous phase of the first arrangement is led via a discharge pipe to a third arrangement, into which the aqueous phase separated in the second arrangement also flows via a discharge pipe for an anaerobic fermentation of the collected aqueous phases, and in which
a) the biogas formed in the first, second and third arrangements is collected in a storage vessel,
b) the sediment formed in the second and third arrangements is removed as sludge or as a mass further processed into compost and
c) the purified waste water formed in the third arrangement is removed after aeration.

3. Method in accordance with claim 1 or 2, characterised in that part of the biogas formed is led through the influent brought into the first arrangement.

4. Method according to claim 3, characterised in that the sludge and water phases formed in the first arrangement are enriched with biogas.

5. Method according to claims 1 to 4, characterised in that the aqueous phase in the third arrangement is brought into agitation by part of the biogas formed.

6. Method according to claims 1 to 5, characterised in that at least part of the sediment formed in the third arrangement is led back as an inoculant into the sludge phase of the second arrangement.

7. Method in accordance with claims 1 to 6, characterised in that the agitation and the separation of sediment in the third arrangement take place in inter-connected compartments separated for this purpose.

8. Installation for the treatment of waste water by anaerobic fermentation in a number of successive operations for the preparation of biogas, sludge and purified waste water, characterised in that the installation consists of a number of arrangements closed off from the air, which are connected to each other by supply and discharge pipes, in which respect each arrangement has means available for promoting a separation of the mass brought up for purification into an aqueous phase and into a sludge phase and the discharge pipe of the aqueous phase of an arrangement leads to a separate arrangement for the anaerobic purification of an aqueous phase, and the discharge pipe of the sludge phase of an arrangement leads to a separate arrangement for the anaerobic fermentation of a sludge phase.

9. Installation in accordance with claim 8, characterised in that the installation consists of arrangements the first of which can be connected to a buffer zone which is furthermore equipped with means for effecting a main separation into an aqueous phase and into a sludge phase and with separate pipes for the removal of biogas formed, for the removal of the sludge phase to a second arrangement for the fermentation of the sludge phase and with a discharge pipe for conveying the aqueous phase to a third arrangement for the anaerobic fermentation of the aqueous phase, into which third arrangement the discharge pipe of the water phase in the second arrangement also flows.

10. Installation in accordance with claim 9, characterised in that means are present for gasifying the sludge phase coming into the second arrangement with the biogas already formed.

11. Installation in accordance with claim 9 or 10, characterised in that the third compartment is constructed in the form of two or more inter-connected compartments, in which respect in the compartment into which the water/aqueous phase is conveyed, means are present for bringing this phase into agitation, and the other compartments from which the purified effluent is removed contain means for promoting the separation of solid parts.

12. Installation in accordance with claim 11, in which a pipe for the transport of the biogas formed runs into the first of the said compartments for the gasification of the water/aqueous phase.

13. Installation in accordance with claim 11 or 12, characterised in that the other compartment(s) referred to contain(s) a discharge pipe for leading the sediment formed to the second arrangement.

FIG. 1

0 223 280

1/1

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | EP-A-0 119 430 (LINDE) * Claims 1-3,5-7; page 2, line 2 - page 3, line 29 * | 1,8,9 | C 02 F 3/28 |
| A | GB-A-2 013 170 (P.J. NEWELL et al.) * Page 5, claims 1,5,6 * | 1 | |
| A | FR-A-2 541 669 (MULTIBIO) * Page 5, line 25 - page 7, line 17 * | 1 | |
| A | FR-A- 926 204 (A. ABDON) * Page 3, abstract * | 1 | |

-----

TECHNICAL FIELDS
SEARCHED (Int. Cl.4)

C 02 F

The present search report has been drawn up for all claims

| Place of search | Date of completion of the search | Examiner |
|---|---|---|
| THE HAGUE | 21-01-1987 | TEPLY J. |